# EUROPÄISCHE PATENTSCHRIFT

(11) **EP 2 399 913 B1**
(45) Veröffentlichungstag und Bekanntmachung des Hinweises auf die Patenterteilung: **18.09.2013**
(21) Anmeldenummer: 10167327.5
(22) Anmeldetag: 25.06.2010
(51) Int. Cl.: C07D 317/64, C07D 493/04, C07D 493/10, C07D 493/14, C09B 57/00

(54) **Fluoreszenzfarbstoff und seine Verwendung**
Fluorescent dye and its application
Colorant fluorescent et son utilisation

(43) Veröffentlichungstag der Anmeldung: 28.12.2011
(73) Patentinhaber: Universität Potsdam, 14469 Potsdam (DE)
(72) Erfinder: Wessig, Pablo, Prof. Dr., 13353 Berlin (DE); Möllnitz, Kristian, 13127 Berlin (DE); Wawrzinek, Robert, 12099 Berlin (DE)
(74) Vertreter: Gulde Hengelhaupt Ziebig & Schneider

(56) Entgegenhaltungen:
- WO-A1-91/12024
- CH-A5- 696 358
- DALLACKER FRANZ ET AL: "Conversion of symmetrically substituted aromatic ethers into benzoquinones", CHEMIKER ZEITUNG, WILEY - V C H VERLAG GMBH & CO. KGAA, DE, Bd. 110, Nr. 10, 1. Januar 1986 (1986-01-01), Seiten 372-373, XP009142534, ISSN: 0009-2894
- DALLACKER FRANZ ET AL: "Derivate des Methylendioxybenzols, 34. Über die Darstellung von Dimethoxy-methylendioxy-benzoldicarbonsäur en", CHEMISCHE BERICHTE, Bd. 104, Nr. 8, 1. August 1971 (1971-08-01), Seiten 2534-2544, XP002614625, ISSN: 0009-2940
- DALLACKER FRANZ ET AL: "Metalation of symmetrically substituted aromatic ethers", CHEMIKER ZEITUNG, WILEY - V C H VERLAG GMBH & CO. KGAA, DE, Bd. 110, Nr. 10, 1. Januar 1986 (1986-01-01), Seiten 369-371, XP009142673, ISSN: 0009-2894
- ORLEMANS ET AL: ""tert-Amino effect" in heterocyclic synthesis. The effect of a p-quinone moiety on the [1,6] H-transfer and 1,5-electrocyclization reactions", JOURNAL OF ORGANIC CHEMISTRY, Bd. 53, Nr. 10, 1. Mai 1988 (1988-05-01), Seiten 2278-2287, XP002614626, DOI: 10.1021/jo00245a029

## Beschreibung

Die Erfindung betrifft einen Fluoreszenzfarbstoff auf Basis eines 1,2,4,5-Tetrahydroxy-benzolderivats mit langer Fluoreszenzlebensdauer und großer Stokes-Verschiebung sowie die Verwendung dieser Verbindung als Fluoreszenzfarbstoff.

Als Fluoreszenzfarbstoffe werden solche Farbstoffe bezeichnet, die bei Bestrahlung mit Licht einer von diesen Substanzen absorbierten Wellenlänge Licht einer (in der Regel) anderen Wellenlänge aussenden. Diese Fluoreszenzfarbstoffe finden vielfältige Anwendung in der Biochemie, Biologie und Medizin, beispielsweise in Diagnostik-Kits, in der Mikroskopie oder beim Wirkstoffscreening.

Aus struktureller Sicht basi-eren Fluoreszenzfarbstoffe zumeist auf aromatischen Strukturen, insbesondere anellierten Heteroaromaten. Eine Gruppe von Fluoreszenzfarbstoffen leitet sich von Xanthen ab, beispielsweise Fluorescin, Calcein, Eosin oder Merbromin. Zu den Xanthen-basierten Farbstoffen zählen auch die Rhodamine, wie Rhodamin B, Rhodamin 6G, Rhodamin 123, Texas Red, Tetramethylrhodaminmethylester (TMRM). Auf einem verwandten Grundgerüst, nämlich Benzophenoxazin, basieren Farbstoffe, wie Nilblau oder Nilrot. Daneben sind Cumarinfarbstoffe bekannt, wie das Cumarin selbst oder Umbelliferon; Indolfarbstoffe, beispielswiese 4',6-Diamidin-2-phenylindol; Phenanthridin-Farbstoffe, wie Ethidiumbromid oder Propidiumiodid; Benzofuran-Farbstoffe, wie Furaptra oder die Fura-Farbstoffe; und Benzothiazol-Farbstoffe, wie Luciferin oder SYBR Green I. Auf verschiedenen anderen heteroaromatischen Grundgerüsten basieren Xylenolorange, Berberin, Epiocconon, IAEDANS oder Coelanterazin. Erwähnenswert sind ferner die Cyanine (Polymethin-Farbstoffe), deren prominenteste Vertreter Indocyaningrün, Cy5 und Cy7 sind.

Fluoreszenzfarbstoffe werden durch eine Reihe von Parametern charakterisiert, die dem Anwender die Auswahl eines geeigneten Farbstoffs abhängig von dem gewünschten Verwendungszweck erlauben. Hier sind vor allem zu nennen die Anregungswellenlänge λ_{exc} (oder λ_{Abs}), welche dem Maximum der Absorptionsbande entspricht, die Emissionswellenlänge λₑₘ, welche dem Maximum der Emissionsbande entspricht, die Stokes-Verschiebung (Stokes-Shift) Δλ, welche der Differenz aus Emissionswellenlänge λₑₘ und Anregungswellenlänge λ_{exc} entspricht, der Extinktionskoeffizient ε, welcher den Anteil der bei der Anregungswellenlänge λ_{exc} absorbierten Strahlung widerspiegelt, die Fluoreszenzquantenausbeute Φ_{F}, welche den Quotienten aus der Anzahl emittierter zu absorbierten Photonen entspricht, und die Fluoreszenzlebensdauer τ_{F}, welche der mittleren Zeit entspricht, in der das Molekül in seinem angeregtem Zustand verharrt, ehe es durch die Emission eines Lichtquants in seinen Grundzustand übergeht.

Wünschenswert, insbesondere in Hinblick auf biologische Anwendungen, sind eine große Anregungswellenlänge λ_{exc}, um mit der Anregungsstrahlung möglichst tief in biologische Proben eindringen zu können, ein großer Extinktionskoeffizient ε, damit möglichst viel des eingestrahlten Lichts absorbiert wird, eine große Stokes-Verschiebung Δλ, um möglichst geringe Wechselwirkungen zwischen der Anregungs- und der Emissionsstrahlung zu beobachten, eine lange Fluoreszenzlebensdauer τ_{F}, um die kurzlebige natürliche Hintergrundfluoreszenz biologischer Gewebe ausblenden zu können, und schließlich eine große Fluoreszenzquantenausbeute Φ_{F}, um ein möglichst hohes Signal-Rausch-Verhältnis zu erzielen.

Bekannte Fluoreszenzfarbstoffe mit einem relativ großen Stokes-Shift (Δλ ≥ 100 nm) weisen typischerweise eine relativ kurze Fluoreszenzlebensdauer (τ_{F} ≤ 5 ns) auf. Umgekehrt beobachtet man bei den bekannten Farbstoffen, die eine lange Fluoreszenzlebensdauer (τ_{F} ≥ 15 ns) aufweisen, gleichzeitig relativ geringe Stokes-Verschiebungen (Δλ ≤ 50 nm). Fluoreszenzfarbstoffe, die gleichzeitig einen großen Stokes-Shift und eine lange Fluoreszenzlebensdauer aufweisen, sind hingegen weitgehend unbekannt.

Dallacker & Sanders (Chemiker-Zeitung, 1986, 110, Nr. 10., S. 369-371) beobachteten in ihren Synthesearbeiten, dass 5,6-Dimethoxy-1,3-benzdioxol-4,7-dicarbaldehyd, 5,6-Dimethoxy-1,3-benzdioxol-4-carbaldehyd in etherischer Lösung sowie Benzol[1,2-d:4,5-d']-bis[1,3]dioxol-4-8-dicarbonsäure in DMSO oder Aceton eine starke Fluoreszenz zeigen.

Der Erfindung liegt die Aufgabe zugrunde, einen Fluoreszenzfarbstoff zur Verfügung zu stellen, der möglichst vielen der vorgenannten Anforderungen entspricht. Insbesondere sollte der Farbstoff gleichzeitig eine lange Fluoreszenzlebensdauer und einen großen Stokes-Shift aufweisen.

Diese Aufgabe wird durch einen Fluoreszenzfarbstoff nach Anspruch 1 gelöst. Weitere bevorzugte Ausgestaltungen des Farbstoffs ergeben sich insbesondere aus den übrigen, in den Unteransprüchen genannten Merkmalen.

Der erfindungsgemäße Fluoreszenzfarbstoff entspricht einer der allgemeinen Formeln I oder II 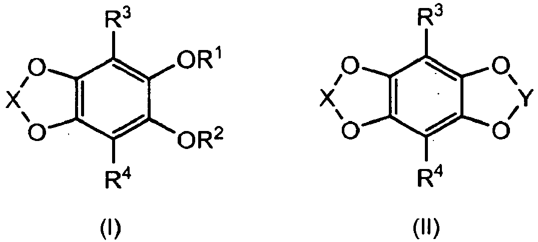 worin R¹, R², R³ und R⁴ unabhängig voneinander Wasserstoff oder einen verzweigten oder unverzweigten, gesättigten oder ungesättigten, aliphatischen oder aromatischen, funktionell substituierten oder unsubstituierten Kohlenwasserstoffrest bedeuten, wobei mindestens einer der Reste R¹ oder R² und einer der Reste R³ oder R⁴ ungleich Wasserstoff ist, wobei zumindest einer der Reste R³ und R⁴, ein C₂- bis C₂₀-Acylrest, ein C₂- bis C₂₀-Acyl-arylrest, ein C₂- bis C₂₀-Acyl-alkenylrest, ein C₂- bis C₂₀-Acyl-alkinylrest oder eine Nitrogruppe ist und wobei in Formel I die Reste R¹ und R³ und/oder die Reste R² und R⁴ miteinander verbrückt sein können, und
X und Y unabhängig voneinander einen substituierten oder unsubstituierten C₁- oder C₂-Kohlenwasserstoffrest bedeuten, wobei eine Kohlenstoffeinheit durch ein N- oder S-Heteroatom ersetzt sein kann.

Eine gleichwertige Definition des erfindungsgemäßen Fluoreszenzfarbstoffs bezieht sich ausschließlich auf die allgemeine Formel I mit den vorstehenden Definitionen der Reste, wobei R¹ und R² miteinander verbrückt sein können.

Formal leitet sich der Fluoreszenzfarbstoff gemäß vorliegender Erfindung von der Struktur IV ab, die sich wiederum von 1,2,4,5-Tetrahydroxybenzol gemäß Formel III ableiten lässt.

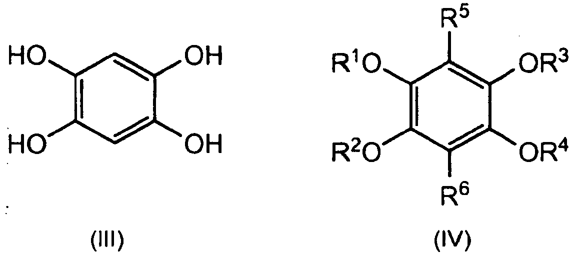

Verbindungen der Struktur IV, bei denen die Reste R¹ bis R⁴ nicht untereinander verbunden (verbrückt) sind und bei denen die Reste R⁵ und R⁶ Elektronenakzeptoren (z. B. Acylgruppen) sind, wurden in der Literatur beschrieben. Diese Verbindungen zeigen keinerlei Fluoreszenz und es finden sich auch keine Hinweise darauf, dass solche Verbindungen als Fluoreszenzfarbstoffe geeignet sein könnten. Die vorliegenden Erfinder haben jedoch festgestellt, dass die Verbrückung eines Paars benachbarter Hydroxygruppen bzw. Alkoxygruppen durch eine Gruppe X (Verbindungen des Typs I) oder beider Paare benachbarter Hydroxygruppen bzw. Alkoxygruppen durch die Gruppen X bzw. Y (Verbindungen des Typs II) zu einer unerwarteten intensiven Fluoreszenz bei gleichzeitig außergewöhnlich großer Stokes-Verschiebung und langer Fluoreszenzlebensdauer führt.

Insbesondere beträgt die Differenz zwischen dem langwelligsten Maximum des Absorptionsspektrums und dem kurzwelligsten Maximum des Emissionsspektrums (Stokes-Verschiebung) zumindest 80 nm, insbesondere zumindest 100 nm, vorzugsweise sogar zumindest 150 nm. Besonders ungewöhnlich ist, dass die erfindungsgemäßen Farbstoffe gleichzeitig eine lange Fluoreszenzlebensdauer von zumindest 5 ns, insbesondere von zumindest 15 ns, vorzugsweise sogar von zumindest 20 ns aufweisen. Vorteilhaft ist ferner ihre große Fluoreszenzintensität, die in Fluoreszenzquantenausbeuten von zumindest 0,2 (20%), insbesondere von zumindest 0,4 (40%), vorzugsweise von zumindest 0,5 (50%), Ausdruck findet.

Vorzugsweise sind in den allgemeinen Formeln I und II die Reste R³ und R⁴ gleich gewählt. Alternativ oder gleichzeitig können in der Struktur gemäß Formel I die Reste R¹ und R² ebenfalls gleich gewählt sein.

Grundsätzlich können für die Reste R¹, R², R³ und R⁴ beliebige Kettenlängen, etwa in Form von Alkyl-, Aryl-, Alkenyl-, Alkinyl-, Alkoxy-, Ether-, Alkoxyether-, Acyl-, Keton-, Carbonsäure-, Carbonsäurester-, Aldehydresten und Mischformen von diesen, vorgesehen sein, insbesondere von bis zu C₅₀-Einheiten, vorzugsweise bis zu C₄₀-Einheiten, noch weiter bevorzugt von bis zu C₃₀-Einheiten, ggf. mit weiteren Heteroatomen und/oder niedermolekularen funktionellen Gruppen. Nach einer vorteilhaften Ausgestaltung können die Reste R¹, R², R³ und R⁴ unabhängig voneinander insbesondere einen verzweigten oder unverzweigten, gesättigten oder ungesättigten, funktionell substituierten oder unsubstituierten C₁-bis C₂₀-Alkylrest, C₅- bis C₂₀-Arylrest, C₂- bis C₂₀-Alkenylrest, C₂- bis C₂₀-Alkinylrest, C₁- bis C₂₀-Alkoxyrest, C₂- bis C₂₀-Etherrest, C₂- bis C₂₀-Alkoxyetherrest, C₂- bis C₂₀-Acylrest, C₃-bis C₂₀-Ketonrest, C₁- bis C₂₀-Carbonsäurerest, C₂- bis C₂₀-Carbonsäuresterrest oder C₁- bis C₂₀-Aldehydrest oder eine Mischform von diesen bedeuten. Vorzugsweise werden diese Reste gewählt aus verzweigten oder unverzweigten, gesättigten oder ungesättigten, funktionell substituierten oder unsubstituierten C₁- bis C₁₅-Alkylresten, C₅- bis C₁₅-Arylresten, C₂- bis C₁₅-Alkenylresten, C₂- bis C₁₅-Alkinylresten, C₁- bis C₁₅-Alkoxyresten, C₂- bis C₁₅-Etherresten, C₂- bis C₁₅-Alkoxyetherresten, C₂- bis C₁₅-Acylresten, C₃- bis C₁₅-Ketonresten, C₁- bis C₁₅-Carbonsäureresten, C₂- bis C₁₅-Carbonsäuresterresten oder C₁- bis C₁₅-Aldehydresten oder Mischformen von diesen. Besonders bevorzugt sind verzweigte oder unverzweigte, gesättigte oder ungesättigte, funktionell substituierte oder unsubstituierte C₁-bis C₁₀Alkylreste, C₅- bis C₁₀-Arylreste, C₂- bis C₁₀-Alkenylreste, C₂- bis C₁₀-Alkinylreste, C₁-bis C₁₀-Alkoxyreste, C₂- bis C₁₀-Etherreste, C₂- bis C₁₀-Alkoxyetherreste, C₂- bis C₁₀-Acylreste, C₃- bis C₁₀-Ketonreste, C₁- bis C₁₀-Carbonsäurereste, C₂- bis C₁₀-Carbonsäuresterreste oder C₁- bis C₁₀-Aldehydreste oder Mischformen von diesen.

Im Falle von R³ und R⁴ sind Acylreste der vorstehenden Definitionen besonders bevorzugt.

Die in den allgemeinen Formeln I oder II optionale Verbrückung der Reste R¹ und R³ und/oder die Reste R² und R⁴ miteinander führt vorzugsweise zu einem 4- bis 7-gliedrigen Ring, insbesondere zu einem 5- bis 6-gliedrigen Ring, wobei ein 6-gliedriger Ring besonders bevorzugt ist (s.u. Farbstoff G).

Als funktionell substituiert wird im Rahmen der vorliegenden Erfindung der Austausch einer oder mehrerer Wasserstoffe der Kohlenwasserstoffreste durch niedermolekulare funktionelle Gruppen verstanden, wie Sulfonsäure-, Sulfhydryl-, Sulfat-, Sulfit-, Phosphonsäure-, Phosphat-, Nitro-, Nitrat-, Nitrit-, Amino-, Amid-, Hydroxy-, Silyl-, Alkoxygruppen und/oder Halogenatome.

Erfindungsgemäß ist in den Strukturen I und II zumindest einer der Reste R³ und R⁴, insbesondere beide Reste R³ und R⁴, ein Elektronenakzeptor, worunter ein Rest verstanden wird, der die Elektronendichte im zentralen Benzolring verringert. Es handelt sich hierbei um eine Nitrogruppe, einen C₂- bis C₂₀-Acylrest, -Acyl-arylrest, -Acyl-alkenylrest, oder -Acyl-alkinylrest, wobei diese Reste noch durch insbesondere "elektronenziehende" Gruppen wie Nitrogruppen substituiert sein können. Besonders bevorzugt ist ein C₂- bis C₁₀-Acylrest,-Acyl-arylrest, -Acyl-alkenylrest, oder -Acyl-alkinylrest. In speziellen Ausführungen sind die Reste R³ und/oder R⁴ ein Methylacylrest, ein Ethylacylrest, ein Propylacylrest, ein tert-Butylacylrest, ein n-Propylacylrest.

Mit Vorteil bedeuten in den allgemeinen Formeln I und II die verbrückenden Reste X und Y unabhängig voneinander einen substituierten oder unsubstituierten C₁-Kohlenwasserstoffrest, insbesondere einen CH₂-Rest, CHR⁵-Rest oder CR⁵R⁶-Rest. Im Allgemeinen können hierbei die Reste R⁵ und R⁶ aus den für die Reste R¹ bis R⁴ vorstehend aufgeführten Gruppen gewählt sein, wobei R⁵ und R⁶ auch miteinander verbrückt sein können, insbesondere zu einem 3- bis 6-atomigen Spirocyclus. In speziellen Ausführungen sind die Reste X und Y als Methylenrest (s.u. Farbstoffe A-C, E, F), als spiro-Cyclohexylrest (s.u. Farbstoff D) oder als 1-Alkoxycarbonyl-1-alkoxycarbonylmethyl-methylen (s.u. Farbstoffe E, F) gewählt.

Bevorzugt kann in der allgemeinen Formel I zumindest einer der Reste R¹ und R², insbesondere beide Reste R¹ und R², ausgewählt sein aus der Gruppe bestehend aus einem C₁- bis C₂₀-Alkoxyrest, C₂- bis C₂₀-Etherrest, C₂- bis C₂₀-Alkoxyetherrest, Insbesondere kommen hier C₁- bis C₁₅-Alkoxyreste, C₂- bis C₁₅-Etherreste, C₂- bis C₁₅-Alkoxyetherreste in Frage. Besonders bevorzugt werden R¹ und/oder R² aus C₁- bis C₁₀-Alkoxyresten, C₂- bis C₁₀-Etherresten, C₂- bis C₁₀-Alkoxyetherresten gewählt.

Besonders bevorzugte Beispiele für erfindungsgemäße Fluoreszenzfarbstoffe entsprechen den Formeln A bis G:

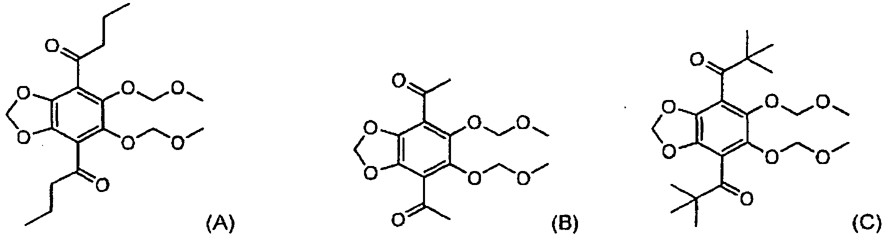

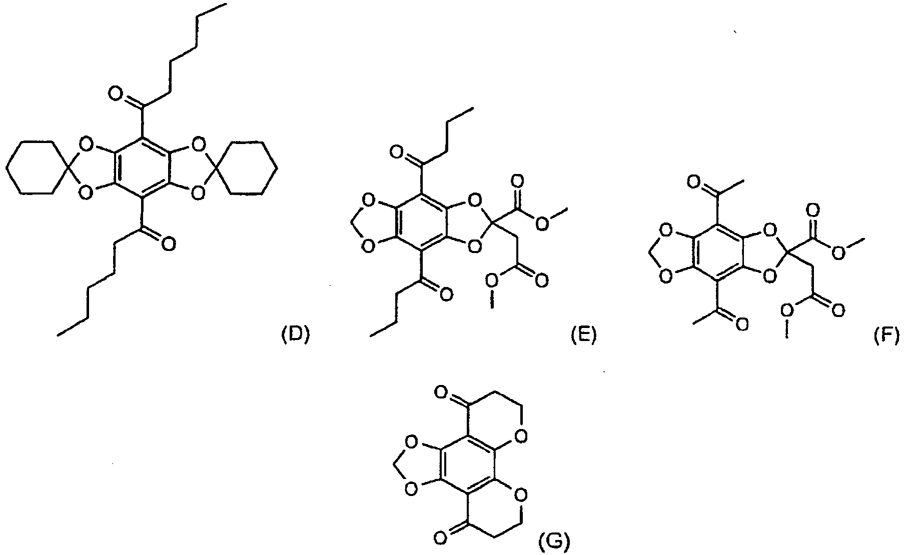

Die vorliegende Erfindung betrifft in einem weiteren Aspekt ein Konjugat umfassend einen erfindungsgemäßen Fluoreszenzfarbstoff nach der vorstehenden Beschreibung und ein an den Fluoreszenzfarbstoff gekoppeltes Biomolekül oder eine an diesen gekoppelte Zelle. Typischerweise kann das Biomolekül ausgewählt sein aus der Gruppe der Proteine, Peptide, Nukleinsäuren, Lipiden und Kohlenhydraten.

Noch ein weiterer Aspekt der vorliegenden Erfindung betrifft die Verwendung der erfindungsgemäßen Verbindung gemäß den allgemeinen Formeln I oder II nach der vorstehenden Beschreibung oder des Konjugats als Fluoreszenzfarbstoff, insbesondere in biologischen oder medizinischen Anwendungen.

Die Erfindung wird nachfolgend in Ausführungsbeispielen anhand der zugehörigen Zeichnungen näher erläutert. Es zeigen:
- Figur 1: Absorptionsspektren und Emissionsspektren von drei erfindungsgemäßen Fluoreszenzfarbstoffen gemäß den Beispielen 1 bis 3 (Farbstoffe A, B und C),
- Figur 2: Absorptionsspektren und Emissionsspektren von drei erfindungsgemäßen Fluoreszenzfarbstoffen gemäß den Beispielen 4 bis 6 (Farbstoffe D, E und F) und
- Figur 3: Korrelation von Stokes-Shift Δλ und Fluoreszenzlebensdauer τ_{F} der erfindungsgemäßen Fluoreszenzfarbstoffe A bis F der Beispiele 1 bis 6 im Vergleich zu einigen bekannten Fluoreszenzfarbstoffen.

Nachfolgend werden beispielhaft die Synthese und die optischen Eigenschaften von sechs Fluoreszenzfarbstoffen A bis F gemäß der vorliegenden Erfindung vorgestellt, wobei die Farbstoffe A, B, C Beispiele für die allgemeine Struktur I und die Farbstoffe D, E, F Beispiele für die allgemeine Struktur II darstellen.

### 1. Beispiel: Fluoreszenzfarbstoff A

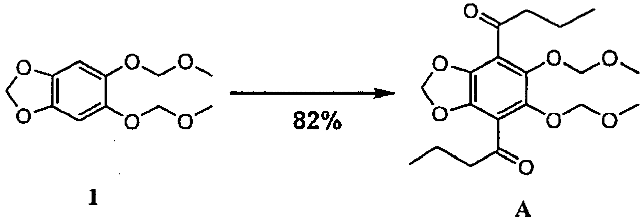

3559 mg (14,69 mmol) der Verbindung **1** wurden in 250 mL trocknem THF vorgelegt. Es wurden 24 mL (38,4 mmol) *n*-Butyl-Lithium (1,6 M) zugetropft und anschließend 7 g (36,76 mmol) Cul hinzugegeben. Der Suspension wurden dann 6,1 mL Buttersäurechlorid zugegeben und bei RT gerührt. Es wurde gesättigte NH₄Cl-Lösung hinzugegeben, mit Et₂O extrahiert, die vereinigten organischen Phasen mit MgSO₄ getrocknet und das Lösungsmittel entfernt. Nach Reinigung mit FC wurden 4628 mg (12,1 mmol, 82%) der Verbindung **A** als gelber Feststoff erhalten.

### 2. Beispiel: Fluoreszenzfarbstoff B

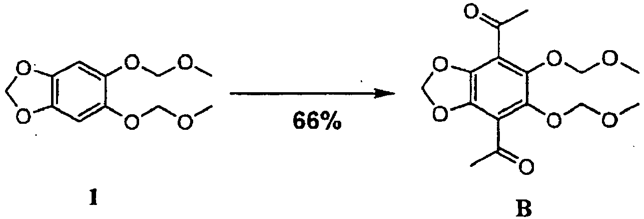

300 mg (1,24 mmol) der Verbindung **1** wurden in 30 mL trocknem THF vorgelegt. Es wurden 2 mL (3,22 mmol) *n-*Butyl-Lithium (1,6 M) zugetropft und anschließend 590 mg (3,1 mmol) Cul hinzugegeben. Der Suspension wurden dann 0,65 mL (5,57 mmol) Acetylchlorid zugetropft und bei RT gerührt. Es wurde gesättigte NH₄Cl-Lösung hinzugegeben, mit Et₂O extrahiert, die vereinigten organischen Phasen mit MgSO₄ getrocknet und das Lösungsmittel entfernt. Nach Reinigung mit FC wurden 265 mg (0,81 mmol, 66%) der Verbindung **B** als gelber Feststoff erhalten.

### 3. Beispiel: Fluoreszenzfarbstoff C

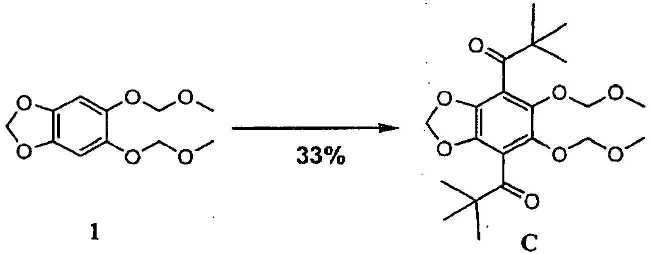

200 mg (0,8 mmol) der Verbindung **1** wurden in 30 mL trocknem THF vorgelegt. Es wurden 1,35 mL (2,16 mmol) *n-*Butyl-Lithium (1,6 M) zugetropft und anschließend 393 mg Cul hinzugegeben. Der Suspension wurden dann 0,46 mL Pivalinsäurechlorid zugetropft und bei RT gerührt. Es wurde gesättigte NH₄Cl-Lösung hinzugegeben, mit Et₂O extrahiert, die vereinigten organischen Phasen mit MgSO₄ getrocknet und das Lösungsmittel entfernt. Nach Reinigung mit FC wurden 112 mg (0,27 mmol, 33%) der Verbindung **C** als weißer Feststoff erhalten.

### 4. Beispiel: Fluoreszenzfarbstoff D

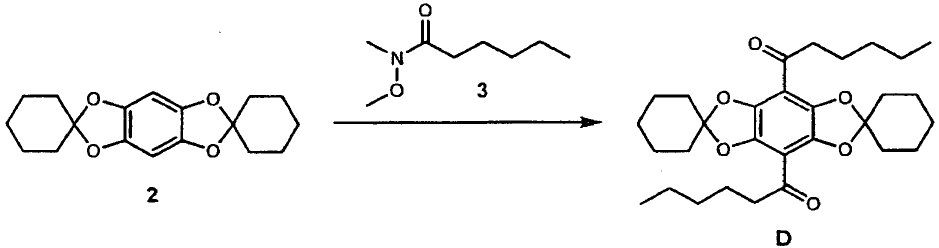

Verbindung **2** (3,69 g, 12,20 mmol) wurde unter Schutzgas in trockenen THF vorgelegt, TMEDA (3,7 mL, 24,8 mmol, 2,0 eq.) und *n-*Butyl-Lithium (16,8 mL, 26,88 mmol, 2,2 eq.) zugegeben. Es wurde das Weinrebamid **3** (3,89 g, 24,43 mmol, 2,0 eq.) zugegeben und mit wässriger Weinsäurelösung gewaschen. Die wässrige Phase wurde mit Diethylether extrahiert und die vereinigten organischen Phasen mit gesättigter NaCl-Lösung gewaschen, über Magnesiumsulfat getrocknet und die Lösungsmittel entfernt. Der erhaltene Rückstand wurde mittels FC gereinigt. Es wurde Verbindung **D** als oranger Feststoff erhalten (0,1 g, 0,20 mmol, 1,6%).

### 5. Beispiel: Fluoreszenzfarbstoff E

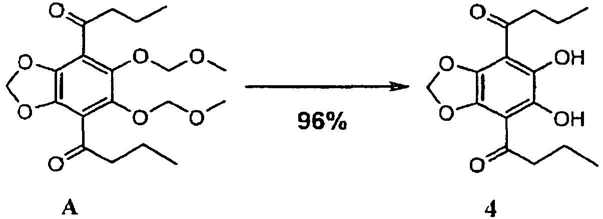

3807 mg (9,96 mmol) von Verbindung **A** aus Beispiel 1 wurden in 80 mL Methanol und 1 mL H₂O gelöst und etwas *p*-Toluolsulfonsäure hinzugegeben. Der ausfallende rote Niederschlag wurde vom Lösungsmittel befreit und mit FC gereinigt. Es wurden 2816 mg (0,48 mmol, 96%) der Verbindung **4** als dunkelrote Kristalle erhalten.

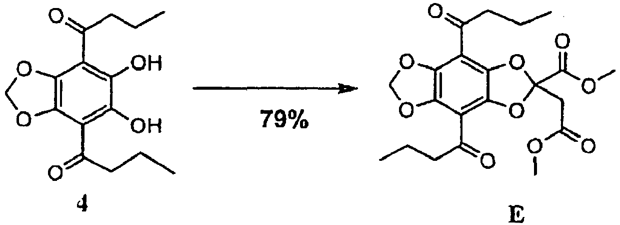

500 mg (0,43 mmol) der Verbindung **4** wurden in 25 mL trockenem Dichlormethan vorgelegt. Eine katalytische Menge 1,4-Diazabicyclo[2.2.2]octan und 241 mg (1,7 mmol) Acetylendicarbonsäuredimethylester wurden hinzugegeben. Nach Beendigung der Reaktion wurden 50 mL 20%-ige Weinsäurelösung zugesetzt. Es wurde mit Et₂O extrahiert und die organischen Phasen mit verdünnter HCl und NaCl-Lösung gewaschen, über MgSO₄ getrocknet und das Lösungsmittel entfernt. Nach Reinigung per FC wurden 582 mg (1,33 mmol, 79%) der Verbindung **E** als orangefarbener Feststoff erhalten.

### 6. Beispiel: Fluoreszenzfarbstoff F

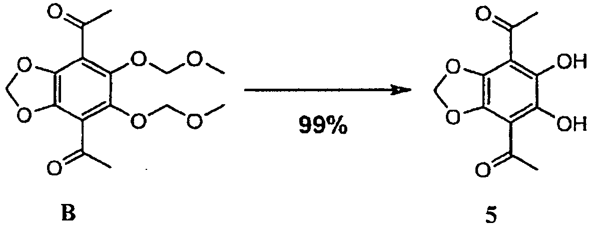

744 mg (2,28 mmol) von Verbindung **B** aus Beispiel 2 wurden in 200 mL Methanol und 1 mL H₂O gelöst und etwas *p*-Toluolsulfonsäure hinzugegeben. Der ausfallende rote Niederschlag wurde vom Lösungsmittel befreit und mit FC gereinigt. Es wurden 538 mg (2,26 mmol, 99%) der Verbindung **5** als dunkelrote Kristalle erhalten.

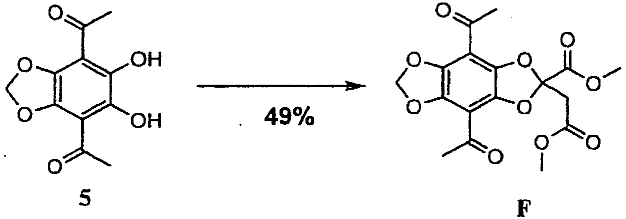

100 mg (0,43 mmol) der Verbindung **5** wurden in 25 mL trockenem Dichlormethan vorgelegt. Eine katalytische Menge 1,4-Diazabicyclo[2.2.2]octan und 73 mg (0,51 mmol) Acetylendicarbonsäuredimethylester wurden hinzugegeben. Nach Beendigung der Reaktion wurden 50 mL 20%-ige Weinsäurelösung zugesetzt. Es wurde mit Et₂O extrahiert und die organischen Phasen mit verdünnter HCl und NaCl-Lösung gewaschen, über MgSO₄ getrocknet und das Lösungsmittel entfernt. Nach Reinigung per FC wurden 80 mg (0,21 mmol, 49%) der Verbindung **F** als orangefarbener Feststoff erhalten.

### 7. Optische Eigenschaften der Verbindungen A-F

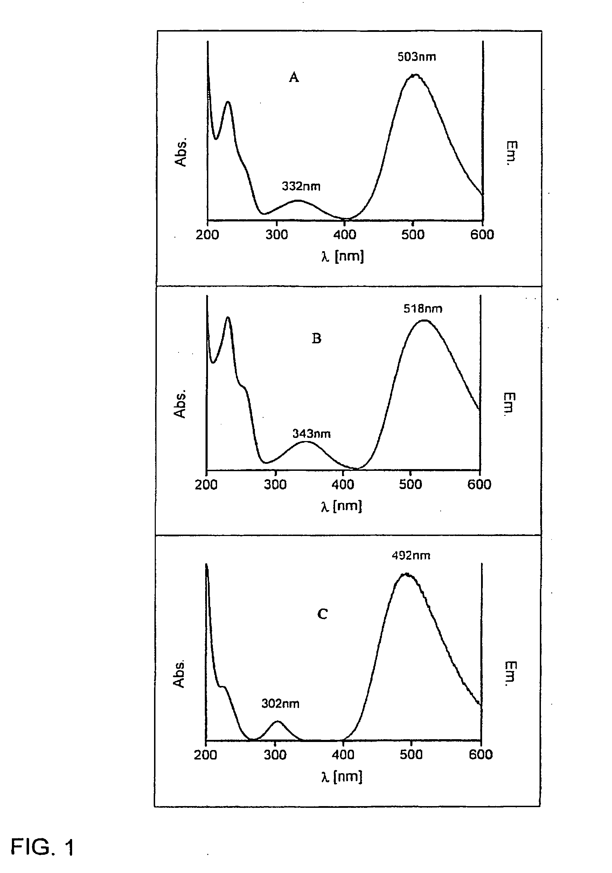

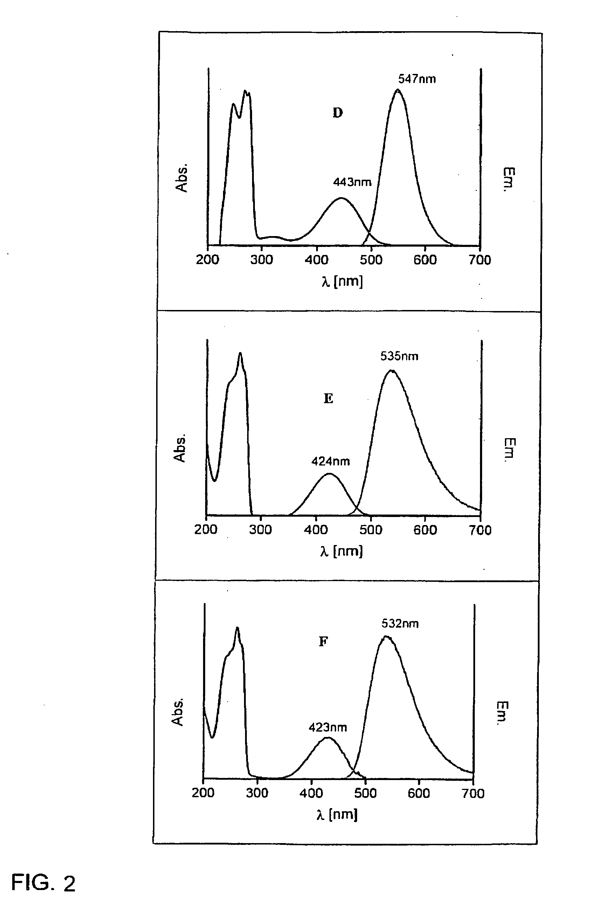

Die Absorptionsspektren und Emissionsspektren der Verbindungen A bis F der Beispiele 1 bis 6 wurden in einem Fluoreszenzspektrometer gemessen. Figur 1 zeigt diese Spektren der Verbindungen A-C und Figur 2 die entsprechenden Spektren der Verbindungen D-F. Aus dem jeweiligen Maximum der Absorptionsspektren und Emissionsspektren wurden die Anregungswellenlänge λ_{abs} beziehungsweise Emissionswellenlänge λₑₘ bestimmt sowie aus der Differenz dieser beiden der Stokes-Shift Δλ berechnet.

Es wurden ferner die Extinktionskoeffizienten ε und die Fluoreszenzquantenausbeuten Φ_{F} gegen geeignete Fluoreszenzstandards ermittelt sowie aus zeitabhängig gemessenen Abklingfunktionen der Fluoreszenz bei λₑₘ die Fluoreszenzlebensdauer τ_{F} ermittelt.

In den Tabellen 1 und 2 sind diese ermittelten Fluoreszenzeigenschaften der Verbindungen A-C beziehungsweise D-F zusammengestellt.

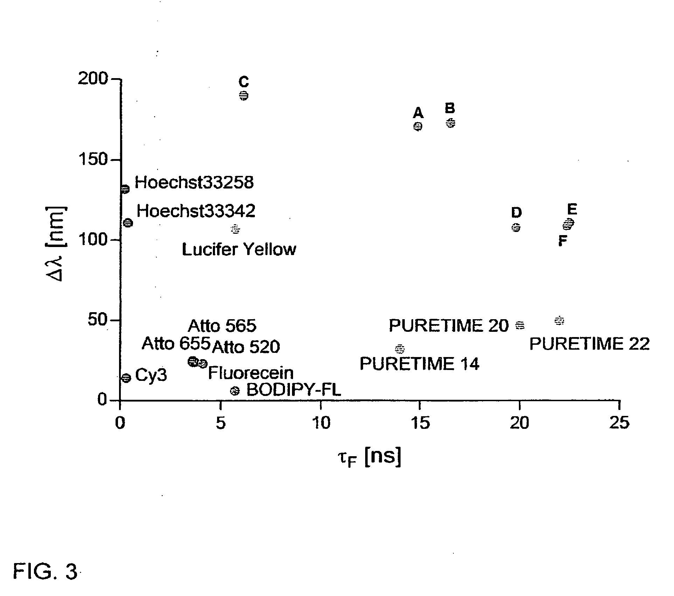

Figur 3 zeigt die Korrelation zwischen dem Stokes-Shift Δλ und der Fluoreszenzlebensdauer τ_{F} für die Verbindungen A-F. Als Vergleich sind die entsprechenden Werte für einige kommerziell erhältliche Fluoreszenzfarbstoffe dargestellt (PURETIME sind Produktbezeichnungen der Fa. AssayMetrics Ltd., 22 Angelica Way, Cardiff, CF14 9FJ, UK; ATTO sind Produktbezeichnungen der Fa. ATTO-TEC GmbH, Am Eichenhang 50, 57076 Siegen; BODIPY sind Produktbezeichnungen der Fa. Invitrogen Corporation, EvoQuest™ Laboratory Services, 5791 Van Allen Way, Carlsbad, CA 92008, USA). Aus der Darstellung ist ersichtlich, dass keiner der bekannten Fluoreszenzfarbstoffe gleichzeitig eine lange Fluoreszenzlebensdauer von τ_{F} ≥ 5 ns und eine große Stokes-Verschiebung von Δλ ≥ 50 nm aufweist. Demgegenüber sind diese beiden Kriterien durch sämtliche der erfindungsgemäßen Fluoreszenzfarbstoffe erfüllt. Insbesondere weisen die drei Fluoreszenzfarbstoffe A-C der allgemeinen Formal I jeweils eine Stokes-Verschiebung von Δλ ≥ 150 nm auf bei einer Fluoreszenzlebensdauer von τ_{F} ≥ 5 ns (für A und B sogar Fluoreszenzlebensdauer von τ_{F} ≥ 15 ns). Umgekehrt zeigen die drei Fluoreszenzfarbstoffe D-F der allgemeinen Formel II jeweils eine Stokes-Verschiebung von Δλ ≥ 100 nm bei einer Fluoreszenzlebensdauer von τ_{F} ≥ 20 ns.

Diese Ergebnisse belegen die herausragenden Fluoreszenzeigenschaften der erfindungsgemäßen Verbindungen, welche ihre besondere Eignung zur Verwendung als Fluoreszenzfarbstoffe zeigen, insbesondere für biologische oder medizinische Anwendungen.

**Tabelle 1: Optische Eigenschaften der Farbstoffe A-C (Lösungsmittel: Acetonitril)**

| Fluorophor | λ_{abs} (nm) | λₑₘ (nm) | Δλ(nm) | ε (M⁻¹cm⁻¹) | τ_{F} (ns) | Φ_{F} | Φ_{F}·ε (M⁻¹cm⁻¹) |
|---|---|---|---|---|---|---|---|
| 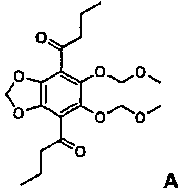 | 332 | 503 | 171 | 2500 | 14,9 | 0,41 | 1030 |
| 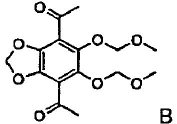 | 343 | 518 | 175 | 1800 | 16,5 | 0,46 | 830 |
| 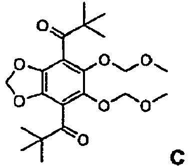 | 302 | 492 | 180 | 3300 | 6,1 | 0,25 | 830 |

**Tabelle 2: Optische Eigenschaften der Farbstoffe D-F (Lösungsmittel: Acetonitril)**

| Fluorophor | λ_{abs}(nm) | λₑₘ(nm) | Δλ(nm) | ε (M⁻¹cm⁻¹) | τ_{F} (ns) | Φ_{F} | Φ_{F}·ε (M⁻¹cm⁻¹) |
|---|---|---|---|---|---|---|---|
| 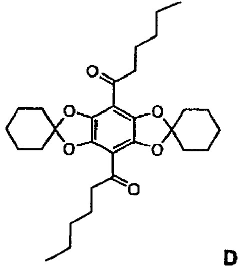 | 443 | 547 | 104 | 4100 | 19.8 | 0.57 | 2300 |
| 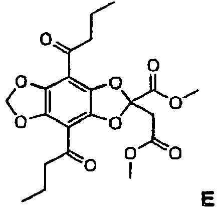 | 424 | 535 | 111 | 4100 | 22.5 | 0.61 | 2500 |
| 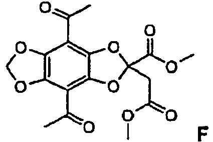 | 423 | 532 | 109 | 4800 | 22.4 | 0.62 | 2980 |

## Patentansprüche

1. Fluoreszenzfarbstoff gemäß der allgemeinen Formel I oderII 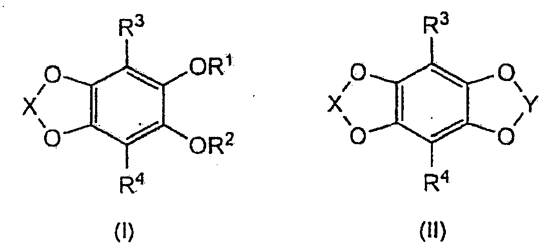 worin R¹, R², R³ und R⁴ unabhängig voneinander Wasserstoff oder einen verzweigten oder unverzweigten, gesättigten oder ungesättigten, aliphatischen oder aromatischen, funktionell substituierten oder unsubstituierten Kohlenwasserstoffrest bedeuten, wobei mindestens einer der Reste R¹ oder R² und einer der Reste R³ oder R⁴ ungleich Wasserstoff ist, wobei zumindest einer der Reste R³ und R⁴, ein C₂- bis C₂₀-Acylrest, ein C₂- bis C₂₀-Acyl-arylrest, ein C₂- bis C₂₀-Acyl-alkenylrest, ein C₂- bis C₂₀-Acyl-alkinylrest oder eine Nitrogruppe ist und wobei in Formel I die Reste R¹ und R³ und/oder die Reste R² und R⁴ miteinander verbrückt sein können und
X und Y unabhängig voneinander einen substituierten oder unsubstituierten C₁- oder C₂-Kohlenwasserstoffrest bedeuten, wobei eine Kohlenstoffeinheit durch ein N- oder S-Heteroatom ersetzt sein kann.

2. Fluoreszenzfarbstoff nach Anspruch 1, wobei die Reste R¹ und R² gleich gewählt sind und/oder die Reste R³ und R⁴ gleich gewählt sind.

3. Fluoreszenzfarbstoff nach einem der vorhergehenden Ansprüche, wobei R¹, R², R³ und R⁴ unabhängig voneinander einen verzweigten oder unverzweigten, gesättigten oder ungesättigten, funktionell substituierten oder unsubstituierten C₁- bis C₂₀-Alkylrest, C₅-bis C₂₀-Arylrest, C₂- bis C₂₀-Alkenylrest, C₂- bis C₂₀-Alkinylrest, C₁- bis C₂₀-Alkoxyrest, C₂- bis C₂₀-Etherrest, C₂- bis C₂₀-Alkoxyetherrest, C₂- bis C₂₀-Acylrest, C₃- bis C₂₀-Ketonrest, C₁- bis C₂₀-Carbonsäurerest, C₂- bis C₂₀-Carbonsäuresterrest oder C₁- bis C₂₀-Aldehydrest bedeuten oder eine Mischform von diesen.

4. Fluoreszenzfarbstoff nach einem der vorhergehenden Ansprüche, wobei beide Reste R³ und R⁴, ein C₂- bis C₂₀-Acylrest, -Acyl-arylrest, -Acyl-alkenylrest, oder -Acyl-alkinylrest oder eine Nitrogruppe sind.

5. Fluoreszenzfarbstoff nach Anspruch 4, wobei zumindest einer der Reste R³ und R⁴, ein C₂- bis C₁₀-Acylrest ist, insbesondere beide Reste R³ und R⁴ ein C₂- bis C₁₀-Acylrest sind.

6. Fluoreszenzfarbstoff nach einem der vorhergehenden Ansprüche, wobei X und Y unabhängig voneinander einen substituierten oder unsubstituierten C₁-Kohlenwasserstoffrest bedeuten, insbesondere einen CH₂-Rest, CHR⁵-Rest oder CR⁵R⁶-Rest, wobei R⁵ und R⁶ miteinander verbrückt sein können, insbesondere zu einem 3- bis 6-atomigen Spirocyclus.

7. Fluoreszenzfarbstoff nach einem der vorhergehenden Ansprüche, wobei zumindest einer der Reste R' und R² ausgewählt ist insbesondere R¹ und R² ausgewählt sind, aus der Gruppe bestehend aus einem C₁- bis C₂₀-Alkoxyrest, C₂- bis C₂₀-Etherrest, C₂- bis C₂₀-Alkoxyetherrest, insbesondere einem C₁- bis C₁₀-Alkoxyrest, C₂- bis C₁₀-Etherrest, C₂-bis C₁₀-Alkoxyetherrest.

8. Fluoreszenzfarbstoff nach einer der Formeln A, B, C oder G:

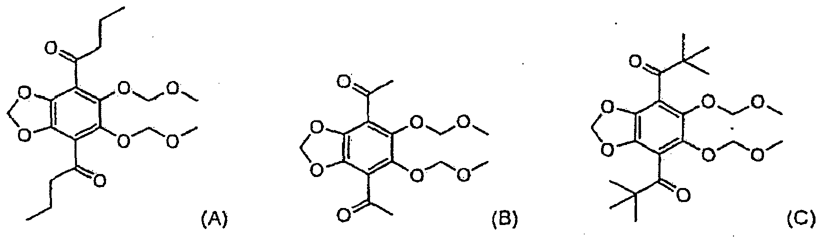

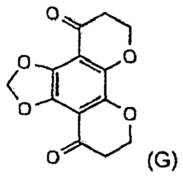

9. Fluoreszenzfarbstoff nach einer der Formeln D, E oder F:

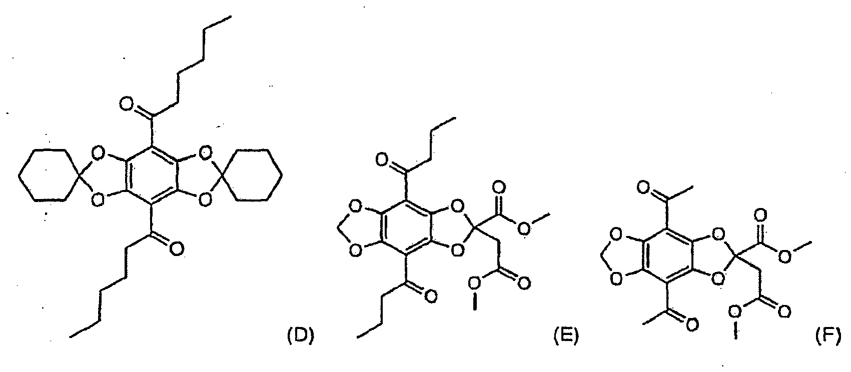

10. Konjugat umfassend einen Fluoreszenzfarbstoff nach einem der Ansprüche 1 bis 9 und ein an den Fluoreszenzfarbstoff gekoppeltes Biomolekül oder eine an den Fluoreszenzfarbstoff gekoppelte Zelle.

11. Konjugat nach Anspruch 10, wobei das Biomolekül ausgewählt ist aus der Gruppe der Proteine, Peptide, Nukleinsäuren, Lipiden und Kohlenhydraten.

12. Verwendung eines Fluoreszenzfarbstoffs nach einem der Ansprüche 1 bis 9 oder eines Konjugats nach einem der Ansprüche 10 bis 11 als Fluoreszenzfarbstoff.

## Claims

1. A fluorescent dye according to general formula I or II: 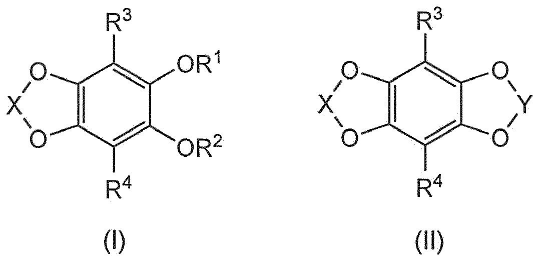 wherein R¹, R², R³ and R⁴ represent independently hydrogen or a branched or unbranched, saturated or unsaturated, aliphatic or aromatic, functionally substituted or unsubstituted hydrocarbon radical, wherein at least one of the R¹ or R² radicals and one of the R³ or R⁴ radicals is not hydrogen, wherein at least one of the R³ and R⁴ radicals is a C₂-C₂₀ acyl radical, a C₂-C₂₀ acylaryl radical, a C₂-C₂₀ acylalkenyl radical, a C₂-C₂₀ acylalkynyl radical or a nitro group, and the R¹ and R³ radicals and/or R² and R⁴ radicals in formula I can be bridged to each other; and
X and Y independently represent a substituted or unsubstituted C₁ or C₂ hydrocarbon radical wherein one carbon unit can be replaced by an N or S heteroatom.

2. The fluorescent dye according to claim 1, wherein the radicals R¹ and R² are selected to be the same and/or the radicals R³ and R⁴ are selected to be the same.

3. The fluorescent dye according to any of the preceding claims, wherein R¹, R², R³ and R⁴ independently represent a branched or unbranched, saturated or unsaturated, functionally substituted or unsubstituted C₁-C₂₀ alkyl radical, C₅-C₂₀ aryl radical, C₂-C₂₀ alkenyl radical, C₂-C₂₀ alkynyl radical, C₁-C₂₀ alkoxy radical, C₂-C₂₀ ether radical, C₂-C₂₀ alkoxyether radical, C₂-C₂₀ acyl radical, C₃-C₂₀ ketone radical, C₁-C₂₀ carboxylic acid radical, C₂-C₂₀ carboxylic ester radical, or C₁-C₂₀ aldehyde radical or a mixed form thereof.

4. The fluorescent dye according to any of the preceding claims, wherein both radicals R³ and R⁴ represent a C₂-C₂₀ acyl radical, a C₂-C₂₀ acylaryl radical, a C₂-C₂₀ acylalkenyl radical or a C₂-C₂₀ acylalkynyl radical or a nitro group.

5. The fluorescent dye according to claim 4, wherein at least one of the radicals R³ and R⁴ is a C₂-C₁₀ acyl radical, in particular both of the radicals R³ and R⁴ are a C₂-C₁₀ acyl radical.

6. The fluorescent dye according to any of the preceding claims, wherein X and Y independently represent a substituted or unsubstituted C₁ hydrocarbon radical, in particular a CH₂ radical, CHR⁵ radical or CR⁵R⁶ radical, and the radicals R⁵ and R⁶ can be bridged to each other to form in particular a 3- to 6-membered spiro ring.

7. The fluorescent dye according to any of the preceding claims, wherein at least one of the radicals R¹ and R² is selected from and in particular both R¹ and R² are selected from the group consisting of a C₁-C₂₀ alkoxy radical, C₂-C₂₀ ether radical, C₂-C₂₀ alkoxyether radical, in particular a C₁-C₁₀ alkoxy radical, C₂-C₁₀ ether radical, C₂-C₁₀ alkoxyether radical.

8. A fluorescent dye according to any of formulas A, B, C or G:

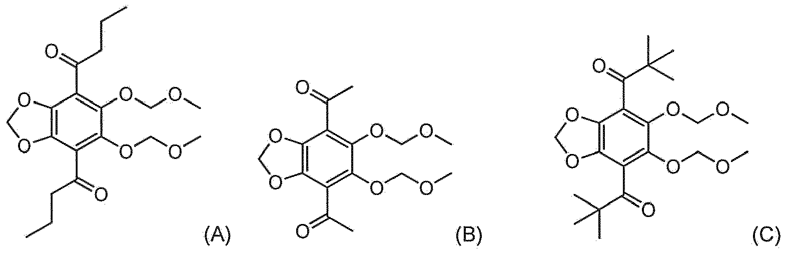

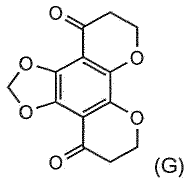

9. A fluorescent dye according to any of formulas D, E or F:

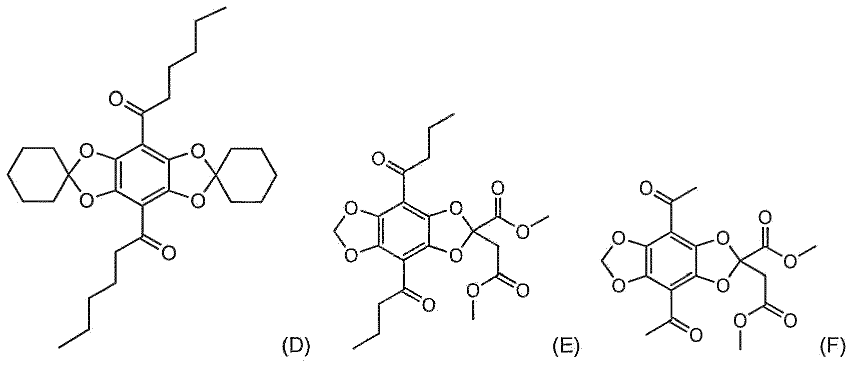

10. A conjugate comprising a fluorescent dye as claimed in any one of claims 1 to 9 and a biomolecule coupled to said fluorescent dye or a cell coupled to said fluorescent dye.

11. The conjugate according to claim 10, wherein the biomolecule is selected from the group of proteins, peptides, nucleic acids, lipids and carbohydrates.

12. Use of a fluorescent dye as claimed in any one of claims 1 to 9 or of a conjugate as claimed in any one of claims 10 to 11 as fluorescent dye.

## Revendications

1. Colorant fluorescent selon la formule générale I ou II 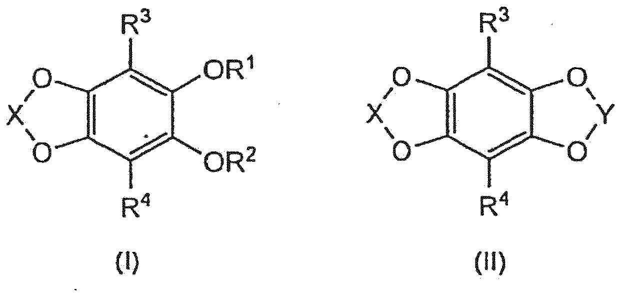 où R¹, R², R³ et R⁴ représentent indépendamment les uns des autres de l'hydrogène ou un radical hydrocarboné ramifié ou non ramifié, saturé ou insaturé, aliphatique ou aromatique, fonctionnellement substitué ou non substitué, au moins un des radicaux R¹ ou R² et un des radicaux R³ ou R⁴ étant différents de l'hydrogène, au moins un des radicaux R³ et R⁴ étant un radical acyle en C₂-C₂₀, un radical acyl-aryle en C₂- C₂₀, un radical acyl-alkényle en C₂-C₂₀, un radical acyl-alkinyle en C₂-C₂₀ ou un groupe nitro et, dans la formule I, les radicaux R¹ et R³ et/ou les radicaux R² et R⁴ pouvant être reliés entre eux par pont et
X et Y représentant indépendamment les uns des autres un radical hydrocarboné en C₁ ou C₂ substitué ou non substitué, une unité carbonée pouvant être remplacée par un hétéroatome N ou S.

2. Colorant fluorescent selon la revendication 1, les radicaux R¹ et R² étant choisis identiques et/ou les restes R³ et R⁴ étant choisis identiques.

3. Colorant fluorescent selon une des revendications précédentes, où R¹, R², R³ et R⁴ représentent indépendamment les uns des autres un radical alkyle en C₁-C₂₀, un radical aryle en C₅-C₂₀, un radical alkényle en C₂-C₂₀, un radical alkinyle en C₂-C₂₀, un radical alcoxy en C₁-C₂₀, un radical éther en C₂-C₂₀, un radical alcoxyéther en C₂-C₂₀, un radical acyle en C₂-C₂₀, un radical cétone en C₃-C₂₀, un radical acide carbonique en C₁-C₂₀, un radical ester d'acide carbonique en C₂-C₂₀ ou un radical aldéhyde en C₁-C₂₀ ramifié ou non ramifié, saturé ou insaturé, fonctionnellement substitué ou non substitué ou un mélange de ceux-ci.

4. Colorant fluorescent selon une des revendications précédentes, les deux radicaux R³ et R⁴ étant un radical acyle en C₂-C₂₀, un radical acyl-aryle en C₂-C₂₀, un radical acyl-alkényle en C₂-C₂₀ ou un radical acyl-alkinyle en C₂-C₂₀ ou un groupe nitro.

5. Colorant fluorescent selon la revendication 4, au moins un des radicaux R³ et R⁴, étant un radical acyle en C₂-C₁₀, en particulier les deux radicaux R³ et R⁴ étant un radical acyle en C₂-C₁₀.

6. Colorant fluorescent selon une des revendications précédentes, X et Y représentant indépendamment l'un de l'autre un radical hydrocarboné C₁ substitué ou non substitué, en particulier un radical CH₂, un radical CHR⁵ ou un radical CR⁵R⁶, R⁵ et R⁶ pouvant être reliés entre eux par pont, en particulier pour donner un cycle spiro à 3 à 6 atomes.

7. Colorant fluorescent selon une des revendications précédentes, au moins un des radicaux R¹ et R² étant sélectionné, en particulier R¹ et R² étant sélectionnés, dans le groupe composé d'un radical alcoxy en C₁-C₂₀, d'un radical éther en C₂-C₂₀, d'un radical alcoxyéther en C₂-C₂₀, en particulier d'un radical alcoxy en C₁-C₁₀, d'un radical éther en C₂-C₁₀, d'un radical alcoxyéther en C₂-C₁₀.

8. Colorant fluorescent selon une des formules A, B, C ou G :

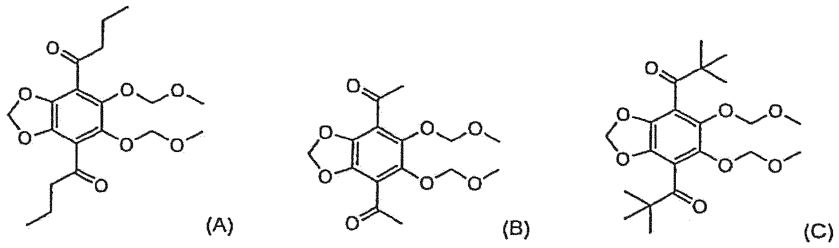

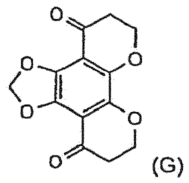

9. Colorant fluorescent selon une des formules D, E, ou F :

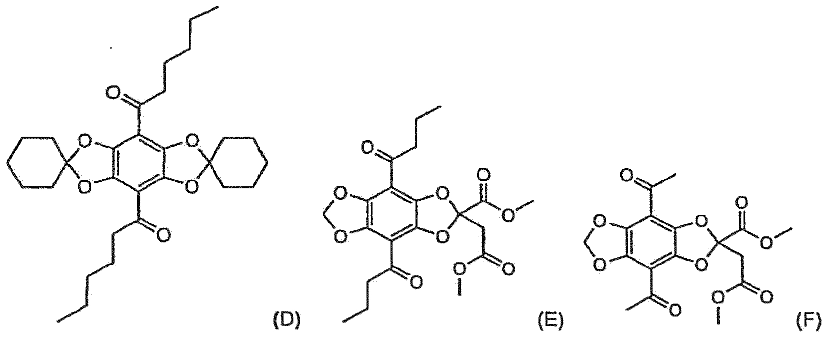

10. Conjugué, comprenant un colorant fluorescent selon une des revendications 1 à 9 et une biomolécule couplée au colorant fluorescent ou une cellule couplée au colorant fluorescent.

11. Conjugué selon la revendication 10, la biomolécule étant sélectionnée dans le groupe des protéines, peptides, acides nucléiques, lipides et glucides.

12. Utilisation d'un colorant fluorescent selon une des revendications 1 à 9 ou d'un conjugué selon une des revendications 10 à 11 en tant que colorant fluorescent.
